# EUROPÄISCHE PATENTANMELDUNG

(11) **EP 3 434 662 A1**
(43) Veröffentlichungstag der Anmeldung: **30.01.2019**
(21) Anmeldenummer: 17183372.6
(22) Anmeldetag: 26.07.2017
(51) Int. Cl.: C07C 2/84, C07C 7/20, C07C 11/02, C07C 11/04, F25J 3/00

(54) **VERFAHREN UND ANLAGE ZUR GEWINNUNG VON OLEFINEN**

(71) Anmelder: Linde AG, 80331 München (DE)
(72) Erfinder: Oberle, David, 81476 München (DE)
(74) Vertreter: m patent group

(57) **Zusammenfassung**

Es wird ein Verfahren (100) zur Herstellung von Olefinen vorgeschlagen, bei dem unter Einsatz eines oder mehrerer stoffumwandelnder Prozesse ein Gasgemisch gebildet wird, das zumindest Methan, Ethan, Ethylen, Kohlenmonoxid und Wasserstoff sowie aromatische Verbindungen, darunter zumindest Benzol, enthält, und das einer Tieftemperaturtrennung (30, 40) zugeführt wird, in welcher es von einem ersten auf ein zweites Temperaturniveau abgekühlt wird. Dabei ist vorgesehen, dass das Gasgemisch vor der Tieftemperaturtrennung (30, 40) an einem oder mehreren Gefrierinhibitoren angereichert wird. Eine entsprechende Anlage ist ebenfalls Gegenstand der vorliegenden Erfindung.

## Beschreibung

Die Erfindung betrifft ein Verfahren zur Gewinnung von Olefinen und eine entsprechende Anlage gemäß den Oberbegriffen der jeweiligen unabhängigen Patentansprüche.

### Stand der Technik

Die oxidative Kopplung von Methan ist in der Literatur beschrieben, beispielsweise bei J.D. Idol et al., "Natural Gas", in: J.A. Kent (Hrsg.), "Handbook of Industrial Chemistry and Biotechnology", Band 2, 12. Auflage, Springer, New York 2012.

Die oxidative Kopplung von Methan umfasst nach derzeitigem Kenntnisstand eine katalysierte Gasphasenreaktion von Methan mit Sauerstoff, bei der von zwei Methanmolekülen jeweils ein Wasserstoffatom abgespalten wird. Die dabei entstehenden Methylradikale reagieren zunächst zu einem Ethanmolekül. Bei der Reaktion wird ferner ein Wassermolekül gebildet. Bei geeigneten Verhältnissen von Methan zu Sauerstoff, geeigneten Reaktionstemperaturen und der Wahl geeigneter Katalysebedingungen erfolgt anschließend eine Oxydehydrierung des Ethans zu Ethylen, einer Zielverbindung bei der oxidativen Kopplung von Methan. Hierbei wird ein weiteres Wassermolekül gebildet. Der eingesetzte Sauerstoff wird bei den genannten Reaktionen typischerweise vollständig umgesetzt.

Die Reaktionsbedingungen bei der oxidativen Kopplung von Methan umfassen klassischerweise eine Temperatur von 500 bis 900 °C, einen Druck von 5 bis 10 bar und hohe Raumgeschwindigkeiten. Jüngere Entwicklungen gehen insbesondere auch in Richtung der Verwendung tieferer Temperaturen. Die Reaktion kann homogen- und heterogenkatalytisch im Festbett oder in der Wirbelschicht erfolgen. Bei der oxidativen Kopplung von Methan können auch höhere Kohlenwasserstoffe mit bis zu sechs oder acht Kohlenstoffatomen gebildet werden, der Schwerpunkt liegt jedoch auf Ethan bzw. Ethylen und ggf. noch Propan bzw. Propylen.

Insbesondere aufgrund der hohen Bindungsenergie zwischen Kohlenstoff und Wasserstoff im Methanmolekül sind die Ausbeuten bei der oxidativen Kopplung von Methan vergleichsweise gering. Typischerweise werden nicht mehr als 10 bis 15% des eingesetzten Methans umgesetzt. Außerdem begünstigen die vergleichsweise harschen Reaktionsbedingungen und Temperaturen, die zur Spaltung dieser Bindungen erforderlich sind, auch die weitere Oxidation der Methylradikale und anderer Intermediate zu Kohlenmonoxid und Kohlendioxid.

Wenngleich den geringen Ausbeuten und der Bildung von Kohlenmonoxid und Kohlendioxid teilweise durch die Wahl optimierter Katalysatoren und angepasster Reaktionsbedingungen entgegengewirkt werden kann, enthält ein bei der oxidativen Kopplung von Methan gebildetes Gasgemisch neben primären Zielverbindungen wie Ethan, Ethylen und ggf. Propylen überwiegend nicht umgesetztes Methan sowie Kohlendioxid, Kohlenmonoxid und Wasser. Ferner sind in dem Gasgemisch auch schwerere Kohlenwasserstoffe wie Aromaten, darunter auch polyzyklische Aromaten, enthalten. Durch ggf. erfolgende nichtkatalytische Spaltreaktionen können auch beträchtliche Mengen an Wasserstoff gebildet werden. Ein derartiges Gasgemisch wird im hier verwendeten Sprachgebrauch auch als "Produktgemisch" der oxidativen Kopplung von Methan bezeichnet, obwohl es überwiegend nicht die gewünschten Produkte, sondern auch das nicht umgesetzte Edukt Methan und die soeben erläuterten Nebenprodukte enthält.

Bei der oxidativen Kopplung von Methan können Reaktoren eingesetzt werden, in denen einer katalytischen Zone eine nichtkatalytische Zone nachgeschaltet ist. Das aus der katalytischen Zone ausströmende Gasgemisch wird in die nichtkatalytische Zone überführt, wo es zunächst noch auf den vergleichsweise hohen Temperaturen vorliegt, die in der katalytischen Zone eingesetzt werden. Insbesondere durch die Anwesenheit des bei der oxidativen Kopplung von Methan gebildeten Wassers ähneln die Reaktionsbedingungen hier jenen herkömmlicher Dampfspaltverfahren (engl. Steam Cracking). Daher können hier Ethan und höhere Paraffine zu Olefinen umgesetzt werden. In die nichtkatalytische Zone können auch weitere Paraffine eingespeist werden, so dass die Restwärme der oxidativen Kopplung von Methan in besonders vorteilhafter Weise ausgenutzt werden kann. Ein derartiges gezieltes Dampfspalten in einer der katalytischen Zone nachgeschalteten nichtkatalytischen Zone wird auch als "Post Bed Cracking" bezeichnet. Nachfolgend wird hierfür auch der Begriff "postkatalytisches Dampfspalten" verwendet.

Bezüglich den beim Dampfspalten herrschenden Reaktionsbedingungen sei ebenfalls auf Fachliteratur wie den Artikel "Ethylene" in Ullmann's Encyclopedia of Industrial Chemistry, Onlineausgabe, 15. April 2007, DOI 10.1002/14356007.a10_045.pub2, verwiesen. Das Dampfspalten wird beispielsweise zur Gewinnung von kurzkettigen Olefinen wie Ethylen und Propylen, Diolefinen wie Butadien oder von Aromaten eingesetzt, ist jedoch nicht hierauf beschränkt.

Nach dem Austritt aus dem Reaktor wird das gebildete Produktgemisch typischerweise einem sogenannten Quench unterworfen, um die ablaufenden Reaktionen möglichst rasch zum Stillstand zu bringen bzw. eine Weiterreaktion zu unerwünschten Nebenprodukten unterbinden. Zu weiteren Details sei auf die zitierte Fachliteratur zum Thema Dampfspalten verwiesen, da der Zweck des Quenchs hier im Wesentlichen derselbe wie bei der oxidativen Kopplung von Methan ist.

Die sich an den Quench anschließende trenntechnische Bearbeitung des Produktgemischs umfasst herkömmlicherweise eine weitere Kühlung auf Umgebungstemperatur. Hierbei kondensiert der überwiegende Teil des in dem Produktgemisch enthaltenen Wassers aus. Die Kühlung erfolgt auf dem Druck, auf dem das Produktgemisch stromab der oxidativen Kopplung von Methan vorliegt, also typischerweise 5 bis 10 bar, beispielsweise 7 bis 8 bar. Anschließend erfolgen eine Verdichtung und danach eine Entfernung des Kohlendioxids, beispielsweise unter Verwendung einer Amin- und/oder Laugenwäsche, aus dem Produktgemisch. Restwasser wird anschließend typischerweise adsorptiv, beispielsweise unter Verwendung von Molsiebadsorbern, abgetrennt.

Ein durch eine entsprechende Aufbereitung aus dem Produktgemisch erhaltenes Gasgemisch, das aber insbesondere noch die genannten schwereren Kohlenwasserstoffe enthält, wird einer Tieftemperaturtrennung zugeführt, in der Methan und tiefer als Methan siedende Komponenten, also insbesondere Kohlenmonoxid und Wasserstoff, sofern enthalten, zumindest überwiegend abgetrennt werden sollen. Das restliche Kohlenwasserstoffgemisch enthält überwiegend oder ausschließlich Ethylen und höher als Ethylen siedende Verbindungen, soweit in dem Produktgemisch enthalten. Bei der Tieftemperaturtrennung wird ein entsprechendes Gasgemisch auf Temperaturen von -60 °C und darunter gekühlt.

Aufgrund dieser tiefen Temperaturen und der spezifischen Zusammensetzung des Gasgemischs wird in den Kondensaten, die in der Tieftemperaturtrennung in den dort eingesetzten Wärmetauschern entstehen, zumindest teilweise der Gefrierpunkt der schwereren Kohlenwasserstoffe unterschritten. Diese, insbesondere Benzol, können sich daher verfestigen ("ausfrieren"). Die gebildeten Feststoffe haften an der wärmeübertragenden Fläche. Die kontinuierliche Ablagerung der Feststoffe führt letztendlich zur Blockierung des Strömungsquerschnitts innerhalb der Wärmetauscher. In der Regel macht dies eine Abstellung des Produktionsbetriebes weit vor dem Erreichen der beabsichtigten Laufzeit erforderlich.

Die naheliegende Problemlösung, die schweren Kohlenwasserstoffe aus dem Gasgemisch zu entfernen, bevor sie in die Tieftemperaturtrennung gelangen, erweisen sich in der Praxis entweder als zu uneffektiv oder sowohl apparativ als auch von der Betriebskostenseite als zu aufwendig.

Die vorliegende Erfindung stellt sich daher die Aufgabe, die Trennung von entsprechenden Gasgemischen, die unter Einsatz der oxidativen Kopplung von Methan gebildet wurden, und die entsprechende schwerere Kohlenwasserstoffe, insbesondere Aromaten, enthalten, zu verbessern.

### Offenbarung der Erfindung

Vor diesem Hintergrund schlägt die vorliegende Erfindung ein Verfahren zur Gewinnung von Olefinen und eine entsprechende Anlage gemäß den Oberbegriffen der jeweiligen unabhängigen Patentansprüche vor. Ausgestaltungen sind jeweils Gegenstand der abhängigen Patentansprüche sowie der nachfolgenden Beschreibung.

Vor der Erläuterung der Merkmale und Vorteile der vorliegenden Erfindung werden noch einige Grundlagen und die verwendeten Begriffe erläutert.

Ist nachfolgend davon die Rede, dass in der Reaktoreinheit ein Anteil des Methans in einem Reaktionseinsatz durch oxidative Kopplung umgesetzt wird, soll hierdurch explizit nicht ausgeschlossen sein, dass die Reaktoreinheit auch zur Durchführung weiterer Verfahren bzw. Verfahrensschritte eingerichtet sein kann, insbesondere das oben erläuterte postkatalytische Dampfspalten. Hierzu können auch separate Reaktoren vorgesehen sein. Entsprechenden weiteren Verfahren oder Verfahrensschritten können weitere Einsätze zugeführt werden, die zumindest teilweise zu Produkten umgesetzt werden, welche sich in einem der Reaktionseinheit entnommenen Produktgemisch wiederfinden. Unter einem "Produktgemisch" wird hierbei das der Reaktionseinheit entnommene Gasgemisch bezeichnet, obwohl dieses nicht nur das oder die gewünschten Produkte sondern ebenso nicht umgesetzte Edukte, insbesondere Methan, und Nebenprodukte enthält. Entsprechendes gilt, wenn hier davon die Rede ist, dass ein Gasgemisch "unter Einsatz" der oxidativen Kopplung von Methan oder eines weiteren stoffumwandelnden Prozesses gebildet wird.

Flüssige und gasförmige Gemische können im hier verwendeten Sprachgebrauch reich oder arm an einer oder mehreren Komponenten sein, wobei "reich" für einen Gehalt von wenigstens 50%, 75%, 90%, 95%, 99%, 99,5%, 99,9% oder 99,99% und "arm" für einen Gehalt von höchstens 50%, 25%, 10%, 5%, 1%, 0,1% oder 0,01% auf molarer, Gewichts- oder Volumenbasis stehen kann. Der Begriff "überwiegend" kann der Definition von "reich" entsprechen.

Die vorliegende Anmeldung verwendet zur Charakterisierung von Drücken und Temperaturen die Begriffe "Druckniveau" und "Temperaturniveau", wodurch zum Ausdruck gebracht werden soll, dass entsprechende Drücke und Temperaturen in einer entsprechenden Anlage nicht in Form exakter Druck- bzw. Temperaturwerte verwendet werden müssen, um das erfinderische Konzept zu verwirklichen. Jedoch bewegen sich derartige Drücke und Temperaturen typischerweise in bestimmten Bereichen, deren Maximal- und Minimalwerte sich um beispielsweise nicht mehr als 1%, 5%, 10%, 20% oder sogar 50% unterscheiden.

Entsprechende Druckniveaus und Temperaturniveaus können dabei in disjunkten Bereichen liegen oder in Bereichen, die einander überlappen. Insbesondere schließen beispielsweise Druckniveaus unvermeidliche oder zu erwartende Druckverluste, beispielsweise aufgrund von Abkühlungseffekten, ein. Entsprechendes gilt für Temperaturniveaus. Bei den hier in bar angegebenen Druckniveaus handelt es sich jeweils um Absolutdrücke.

Bei einer "Rektifikationskolonne" handelt es sich im hier verwendeten Sprachgebrauch um eine Trenneinheit, die dafür eingerichtet ist, ein gasförmig oder flüssig oder in Form eines Zweiphasengemischs mit flüssigen und gasförmigen Anteilen, ggf. auch im überkritischen Zustand, eingespeistes Stoffgemisch durch Rektifikation zumindest teilweise aufzutrennen, also aus dem Stoffgemisch jeweils Reinstoffe oder zumindest Stoffgemische mit anderer Zusammensetzung zu erzeugen.

Typischerweise sind Rektifikationskolonnen als zylindrische Metallbehälter ausgebildet, die mit Einbauten, beispielsweise Siebböden oder geordneten oder ungeordneten Packungen, ausgerüstet sind. Neben den zur eigentlichen Trennung vorgesehenen Bereichen, die nachfolgend auch als "Trennbereiche" bezeichnet werden, können Rektifikationskolonnen auch für andere Zwecke vorgesehene Bereiche aufweisen, beispielsweise Kondensationsräume, die lediglich zur Phasentrennung von Zweiphasengemischen eingerichtet sind und auf diese Weise im Wesentlichen einen entsprechenden extern ausgebildeten Behälter ersetzen.

Eine Rektifikationskolonne weist einen Sumpfverdampfer auf. Hierbei handelt es sich um eine Einrichtung mit einem Wärmetauscher, der beheizt wird, und die dafür eingerichtet ist, eine im Sumpf der Rektifikationskolonne anfallende flüssige Fraktion, auch als Sumpfflüssigkeit bzw. Sumpfprodukt bezeichnet, zu erwärmen. Mittels eines Sumpfverdampfers wird kontinuierlich ein Teil des Sumpfprodukts verdampft und gasförmig in dem Trennbereich zurückgespeist werden.

### Vorteile der Erfindung

Die vorliegende Erfindung betrifft ein Verfahren zur Herstellung von Olefinen, bei dem unter Einsatz eines oder mehrerer stoffumwandelnder Prozesse, insbesondere einer oxidativen Kopplung von Methan, ein Gasgemisch gebildet wird, das zumindest Methan, Ethan, Ethylen, Kohlenmonoxid und Wasserstoff sowie aromatische Verbindungen, darunter Benzol, enthält. Dieses Gasgemisch ist insbesondere arm an oder frei von Kohlendioxid und Wasser, da diese Komponenten bereits, wie oben erläutert, stromauf aus einem Produktgemisch, das unter Einsatz des stoffumwandelnden Prozesses, insbesondere der oxidativen Kopplung von Methan, gebildet wurde, abgetrennt wurden. Das Gasgemisch wird anschließend einer Tieftemperaturtrennung zugeführt. Entsprechende Verfahren sind dem Grundsatz nach dem Fachmann bekannt.

Wie erwähnt, handelt es sich bei dem stoffumwandelnden Prozess insbesondere um eine oxidative Kopplung von Methan, auf die vorstehend vornehmlich Bezug genommen wurde und nachfolgend Bezug genommen wird. Jedoch kann die vorliegende Erfindung grundsätzlich auch im Zusammenhang mit anderen stoffumwandelnden Verfahren zum Einsatz kommen, in denen Gasgemische gleicher oder zumindest vergleichbarer Zusammensetzung gebildet werden, beispielsweise Dampfspaltverfahren, und die in entsprechender Weise einer Tieftemperaturtrennung unterworfen werden. Bei einem Dampfspaltverfahren kann dabei insbesondere ein bekanntes "Demethanizer First"- bzw. "Frontend Demethanizer"-Verfahren zur Trennung des Gasgemischs eingesetzt werden.

Wie bereits oben erwähnt, werden das Gasgemisch oder Anteile des Gasgemischs in der Tieftemperaturtrennung auf ein Temperaturniveau von -60 °C und darunter, insbesondere auf ein Temperaturniveau von -80 °C un d darunter, beispielsweise auf ein auf ein Temperaturniveau von -90 °C und darunte r, abgekühlt und dabei partiell kondensiert. Entsprechende Temperaturniveaus können beispielsweise bei -60 bis - 100 °C oder -80 bis -90 °C liegen und insbesondere in Bereichen, wie sie in der Demethanisierung in bekannten Dampfspaltverfahren eingesetzt werden. Ein entsprechendes Temperaturniveau wird hier auch als "zweites" Temperaturniveau bezeichnet. Das Gasgemisch wird in der Tieftemperaturtrennung auf dieses zweite Temperaturniveau ausgehend von einem Temperaturniveau, das beispielsweise der Umgebungstemperatur entspricht oder darüber liegt, und das hier als "erstes" Temperaturniveau bezeichnet wird, abgekühlt. Allgemein kann das erste Temperaturniveau bei 0 bis 100 °C, insbesondere bei 10 bis 80 °C, beispielsweise bei 20 bis 60 °C oder 30 bis 50 °C liegen.

Wie erwähnt, kann auf den tiefen Temperaturniveaus, auf die das Gasgemisch in der Tieftemperaturtrennung abgekühlt und folglich dabei kondensiert wird, der Gefrierpunkt schwererer in dem Gasgemisch enthaltener Kohlenwasserstoffe, insbesondere von aromatischen Verbindungen wie Benzol, das in einem Gehalt von bis zu 2.000 ppm, insbesondere bis zu 500 ppm, beispielsweise von 200 bis 1.000 ppm (jeweils auf Gewichtsbasis), in einem entsprechenden Gasgemisch enthalten sein kann, unterschritten, so dass es zu den erläuterten Problemen kommen kann. Die in dem Gasgemisch enthaltenen aromatischen Verbindungen umfassen, mit anderen Worten, aromatische Verbindungen, die einen Schmelzpunkt aufweisen, der oberhalb des zweiten Temperaturniveaus liegt.

Die vorliegende Erfindung sieht daher vor, dem Gasgemisch vor der Tieftemperaturtrennung an einem oder mehreren Gefrierinhibitoren anzureichern, also diesen oder diese stromauf der Tieftemperaturtrennung zuzuspeisen. Die Zuspeisung erfolgt insbesondere auf dem ersten Temperaturniveau. Ein im Rahmen der vorliegenden Erfindung eingesetzter Gefrierinhibitor ist vorteilhafterweise bereits in dem Gasgemisch enthalten. Bevorzugt sind dabei beispielsweise Kohlenwasserstoffverbindungen mit einem Schmelzpunkt, der unterhalb von -80 °C, vorzugsweise unterhalb von -90 °C, beispielsweise von bis zu -100 °C, liegt. Im Gegensatz dazu liegt der Schmelzpunkt von Benzol bei 5,5 °C. Ferner sind Kohlenwasserstoffverbindungen bevorzugt, die gute Lösungseigenschaften, insbesondere für höher schmelzende aromatische Verbindungen, aufweisen. Entsprechende Verbindungen frieren daher auf dem zweiten Temperaturniveau nicht aus und bewirken, dass auch die höher siedenden aromatischen Verbindungen, insbesondere Benzol, nicht ausfrieren können (Schmelzpunktabsenkung).

Allgemein wird daher im Rahmen dieser Anmeldung unter einem "Gefrierinhibitor" eine Substanz verstanden, die bei Mischung mit einer sich auf einem Temperaturniveau oberhalb des zweiten Temperaturniveaus verfestigenden Substanz, insbesondere einer aromatischen Verbindung wie Benzol, durch eine Schmelzpunktabsenkung diese Verfestigung verhindert.

Die vorliegende Erfindung ermöglicht es, mit Hilfe eines relativ kleinen apparativen Aufwands bei verhältnismäßig geringen Energie- und Chemikalienkosten die Feststoffbildung in der Tieftemperaturtrennung zu verhindern. Dadurch ergibt sich für die Tieftemperaturtrennung eine sehr hohe Laufzeit ohne signifikante Beeinträchtigung des Produktionsbetriebs.

Entsprechende Gefrierinhibitoren sind unter den gewählten Betriebsbedingungen vorzugsweise reaktionsträge, so dass sie keine unerwünschten Reaktionen mit den anderen, in dem Gasgemisch enthaltenen Verbindungen eingehen bzw. nicht zur Dimerisierung bzw. Polymerisierung neigen. Ferner sollten entsprechende Gefrierinhibitoren vorzugsweise einen möglichst hohen Abstand zwischen Schmelz- und Siedepunkt aufweisen, wodurch sie sich in einem breiten Temperaturbereich in flüssigem Zustand befinden.

Vorzugsweise ist bzw. sind der oder die Gefrierinhibitoren, mit dem oder denen das Gasgemisch vor der Tieftemperaturtrennung im Rahmen der vorliegenden Erfindung angereichert wird, aus zyklischen Kohlenwasserstoffverbindungen, insbesondere aromatischen Verbindungen, ausgewählt. Beispielsweise können Alkylbenzole, insbesondere einfache Alkylbenzole, also solche mit genau einem Alkylrest am Benzolring, verwendet werden, wobei der Alkylrest insbesondere einen, zwei oder drei Kohlenstoffatome umfassen kann. Exemplarische Vertreter sind Toluol, Ethylbenzol, n-Propylbenzol, Cumol und n-Butylbenzol. Auch alizyklische Verbindungen mit beispielsweise drei, vier oder fünf Kohlenstoffatomen wie Cyclopropan, Cyclobutan, Cyclopentan und entsprechende ungesättigte Verbindungen wie Cyclopenten und Cyclopentadien können eingesetzt werden. Alternativ zu diesen zyklischen Kohlenwasserstoffverbindungen können auch mittelschwere nichtzyklische Kohlenwasserstoffe aus den Gruppen der Alkane oder Alkene mit drei bis fünf Kohlenstoffatomen, wie z.B. n-Pentan und n-Butan bzw. Propylen, verwendet werden. Die vorliegende Erfindung wird nachfolgend insbesondere unter Bezugnahme auf Toluol erläutert, wobei die entsprechenden Erläuterungen jedoch andere aromatische, alizyklische und nichtzyklische Kohlenwasserstoffe in gleicher bzw. entsprechender Weise betreffen.

Die Verwendung von Toluol stellt eine sehr vorteilhafte Lösung dar, da Toluol sämtliche der genannten Prozessanforderungen gut erfüllt und destillativ in hoher Konzentration in einem entsprechenden Verfahren wiedergewonnen werden kann (siehe auch unten). Toluol liegt in verhältnismäßig großen Mengen in einem entsprechenden Gasgemisch vor und ist auch als Basischemikalie aus externen Quellen problemlos verfügbar.

Vorzugsweise wird bzw. werden der eine oder die mehreren Gefrierinhibitoren, mit dem oder denen das Gasgemisch vor der Tieftemperaturtrennung im Rahmen der vorliegenden Erfindung angereichert wird, ferner in der Tieftemperaturtrennung oder einem sich der Tieftemperaturtrennung anschließenden Trennschritt aus dem Gasgemisch, das mit ihnen angereichert wurde, oder einer in der Tieftemperaturtrennung oder dem anschließenden Trennschritt gebildeten Fraktion abgetrennt. Auf diese Weise kann ein entsprechender Gefrierinhibitor rezykliert werden und erneut für die erfindungsgemäße Aufgabe zum Einsatz kommen.

Typischerweise wird dabei unter Verwendung zumindest eines Teils des Gasgemischs oder zumindest eines Teils einer in der Tieftemperaturtrennung gebildeten Fraktion ein Komponentengemisch gebildet, das neben dem oder den Gefrierinhibitoren, mit dem oder mit denen das Gasgemisch vor der Tieftemperaturtrennung angereichert wird, überwiegend oder ausschließlich Kohlenwasserstoffe mit drei und mehr Kohlenstoffatomen umfasst. Auf diese Weise kann bzw. können der bzw. die verwendete(n) Gefrierinhibitor(en) einfach rückgewonnen werden, da die Bildung eines entsprechenden Komponentengemischs mit drei und mehr Kohlenstoffatomen üblicherweise bei der Trennung ohnehin vorgenommen wird.

Insbesondere kann bzw. können der bzw. die Gefrierinhibitoren, mit dem oder denen das Gasgemisch vor der Tieftemperaturtrennung angereichert wird, destillativ aus dem genannten Komponentengemisch oder einem Teil hiervon abgetrennt werden. Eine entsprechende Abtrennung ist besonders vorteilhaft, da in dieser nur vergleichsweise geringe Mengen bearbeitet werden müssen. Wie erwähnt, werden bei der oxidativen Kopplung von Methan überwiegend Kohlenwasserstoffe mit zwei Kohlenstoffatomen gebildet, schwerere Kohlenwasserstoffe hingegen nur in vergleichsweise geringeren Mengen. Entsprechendes gilt auch beispielsweise für die Dampfspaltung leichterer, d.h. ethanreicher, Einsätze.

Ein zusätzlicher Vorteil des Einsatzes einer entsprechenden destillativen Trennung ist der, dass aus dem genannten Komponentengemisch auf diese Weise zusätzliche Nebenprodukte, beispielsweise zunächst in Form einer Fraktion, die überwiegend oder ausschließlich Kohlenwasserstoffe mit drei Kohlenstoffatomen umfasst, einer Fraktion, die überwiegend oder ausschließlich Kohlenwasserstoffe mit vier Kohlenstoffatomen umfasst und einer Fraktion, die überwiegend oder ausschließlich Kohlenwasserstoffe mit fünf Kohlenstoffatomen umfasst, oder entsprechender kombinierter Fraktionen gebildet werden. Diese können ohne Mehraufwand zu einer weiteren Wertschöpfung innerhalb des gesamten Verfahrens beitragen.

In Abhängigkeit von der Wahl des oder der Gefrierinhibitoren, mit der oder denen das Gasgemisch vor der Tieftemperaturtrennung angereichert wird, und der Anforderung an die Reinheit können im Rahmen der vorliegenden Erfindung unterschiedliche Trennsequenzen zur Gewinnung vorteilhaft sein. Es können sowohl ein- als auch zwei- oder mehrstufige destillative Verfahren eingesetzt werden. Auch eine Gewinnung durch einen Seitenabzug aus einer oder mehreren in dem destillativen Verfahren eingesetzten Rektifikationskolonne ist beispielsweise möglich.

Um Schwankungen in der Zusammensetzung des der Tieftemperaturtrennung unterworfenen Gasgemischs, insbesondere dem Verhältnis von Benzol zu dem oder den Gefrierinhibitoren, entgegenzuwirken, kann das Einspeisen einer geringen Menge des oder der Gefrierinhibitoren in (möglichst) reiner Form von einer externen Quelle von Vorteil sein. Auch eine zusammensetzungsabhängige Zudosierung, die beispielsweise dynamisch an schwankende Gehalte in dem Gasgemisch angepasst werden kann, ist im Rahmen der vorliegenden Erfindung vorteilhafterweise möglich.

Das vor der Einspeisung des oder der Gefrierinhibitoren vorliegende Gasgemisch weist als Hauptkomponenten insbesondere die folgenden Verbindungen auf:

| **Komponente** | **Gehalt (Molprozent)** |
|---|---|
| Wasserstoff | 4 bis 20 |
| Argon | 0,2 bis 2 |
| Stickstoff | 0 bis 10 |
| Kohlenmonoxid | 1 bis 4 |
| Methan | 50 bis 90 |
| Ethylen | 2 bis 10 |
| Ethan | 1 bis 5 |
| C3-Kohlenwasserstoffe | 0,2 bis 2 |
| C4-Kohlenwasserstoffe | 0,02 bis 0,2 |
| C5-Kohlenwasserstoffe | 0,01 bis 0,1 |
| Benzol | 0,02 bis 0,2 |
| Toluol | 0 bis 0,02 |

Nichtaromatische Kohlenwasserstoffe mit sechs und sieben Kohlenstoffatomen sowie Kohlenwasserstoffe mit acht und mehr Kohlenstoffatomen können zumindest in Spuren ebenfalls enthalten sein.

Die vorliegende Erfindung betrifft auch eine Anlage zur Herstellung eines Olefins mit den Merkmalen des entsprechenden Patentanspruchs.

Zu weiteren Merkmalen und Vorteilen einer entsprechenden Anlage und deren vorteilhaften Eigenschaften sei auf die zuvor erläuterten Verfahrensmerkmale ausdrücklich verwiesen. Dies gilt auch für die vorteilhafterweise vorgesehene Anlage, die zusätzlich zur Durchführung eines Verfahrens eingerichtet ist, wie es zuvor erläutert wurde, und die hierzu eingerichtete Mittel aufweist.

Die Erfindung wird in bevorzugten Ausgestaltungen unter Bezugnahme auf die beigefügten Zeichnungen näher erläutert.

### Kurze Beschreibung der Zeichnung

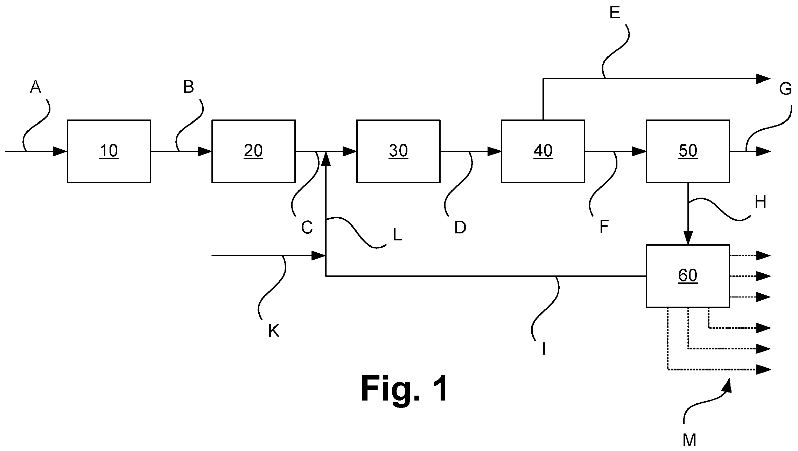

Figur 1 veranschaulicht ein Verfahren gemäß einer Ausführungsform der Erfindung in Form eines vereinfachten Prozessflussdiagramms.

### Ausführliche Beschreibung der Zeichnung

Die Figur 1 veranschaulicht ein Verfahren gemäß einer Ausführungsform der Erfindung in Form eines stark vereinfachten Prozessflussdiagramms. Das Verfahren ist insgesamt mit 100 bezeichnet. Die zu Figur 1 erläuterten Komponenten bzw. Verfahrensschritte sind gleichzeitig Teil entsprechender Anlagen bzw. dort vorrichtungsmäßig implementiert, so dass die nachfolgenden Erläuterungen bezüglich Verfahrensschritten auch eine entsprechende Anlage betreffen und umgekehrt.

In dem Verfahren 100 wird eine oxidative Kopplung von Methan in einer Reaktionseinheit 10 durchgeführt, die einen oder mehrere Reaktoren umfassen kann, wie hier nicht im Detail veranschaulicht. Der oxidativen Kopplung von Methan können weitere Verfahren bzw. Verfahrensschritte zu- bzw. nachgeordnet sein, wie einleitend erläutert und nicht gesondert dargestellt. Wie erwähnt, kann das vorliegend erläuterte Verfahren grundsätzlich auch im Zusammenhang mit anderen stoffumwandelnden Prozessen zum Einsatz kommen.

Die Reaktionseinheit 10 kann insbesondere mit einem oder mehreren von extern zugeführten methanreichen Stoffströmen A sowie mit einem oder mehreren aus den Verfahren 100 rückgeführten methanreichen Stoffströmen gespeist werden, wobei letzteres nicht gesondert veranschaulicht ist. Der Reaktionseinheit 10 können ferner ein oder mehrere sauerstoffreiche Stoffströme zugeführt werden, wie hier ebenfalls nicht gesondert dargestellt. Wird ein eingangs erläutertes postkatalytisches Dampfspalten eingesetzt, können diesem ein oder mehrere paraffinreiche Stoffströme, beispielsweise ein oder mehrerer Ethan- oder Propanströme, zugeführt werden. Auch diese können in den Verfahren 100 gebildet oder von extern zugeführt werden.

Unter Einsatz der oxidativen Kopplung von Methan wird durch die Reaktionseinheit 10, d.h. auch ggf. unter Einsatz weiterer Verfahren bzw. Verfahrensschritte, ein Produktgemisch bereitgestellt, das dessen übliche Komponenten, zumindest aber Methan, Ethan, Ethylen, Kohlenmonoxid und Wasserstoff sowie aromatische Verbindungen, darunter Benzol, enthält. Das Produktgemisch enthält stromab der Reaktionseinheit 10 auch noch Kohlendioxid und Wasser. Es wird in Form eines Stoffstroms B aus der Reaktionseinheit 10 ausgeführt. Das Produktgemisch wird, wie in Figur 1 nicht gesondert dargestellt, einem Quench unterworfen, um dieses rasch abzukühlen und Weiterreaktionen zu unterbinden, und anschließend einer Verdichtung, Kohlendioxidentfernung und Trocknung, wie hier vereinfacht mit einem Block 20 veranschaulicht, unterworfen. Details wurden bereits oben erläutert und sind dem Fachmann, beispielsweise aus dem Bereich der Dampfspaltverfahren, bekannt.

Das entsprechend behandelte Gasgemisch, nun in Form eines Stoffstroms C veranschaulicht, liegt beispielsweise bei Umgebungstemperatur oder allgemeiner bei dem zuvor erläuterten "ersten" Temperaturniveau vor und wird einer Tiefkühlung 30 zugeführt, die insbesondere Teil einer sich anschließenden Tieftemperaturtrennung 40 sein kann. In der Tiefkühlung 30 wird das Gasgemisch auf ein Temperaturniveau von beispielsweise -70 °C und darunter, allgemein auf d as zuvor erläuterte "zweite" Temperaturniveau abgekühlt. Zuvor wird es mit einem Stoffstrom L vereinigt und auf diese Weise mit einem oder mehreren Gefrierinhibitoren, insbesondere Toluol, angereichert, wie bereits oben erläutert.

In der Tieftemperaturtrennung 40, der insbesondere ein oder mehrere gasförmige und/oder flüssige Stoffströme D, insbesondere auch in Form von Zweiphasengemischen zugeführt wird, werden im Rahmen einer grundsätzlich bekannten Demethanisierung eine Fraktion, die überwiegend oder ausschließlich Methan sowie Kohlenmonoxid und ggf. Wasserstoff enthält, und eine Fraktion, die überwiegend oder ausschließlich Kohlenwasserstoffe mit zwei und mehr Kohlenstoffatomen enthält, gebildet. Erstere wird in Form eines Stoffstroms E aus der Tieftemperaturtrennung 40 abgezogen, wobei enthaltenes Methan insbesondere in die Reaktionseinheit 10 zurückgeführt werden kann. Letztere wird in Form eines Stoffstroms F einem weiteren Trennschritt 50 zugeführt, in dem eine Fraktion, die überwiegend oder ausschließlich Kohlenwasserstoffe mit zwei Kohlenstoffatomen gebildet und in Form eines Stoffstroms G abgezogen werden kann.

Es verbleibt eine Fraktion, die überwiegend oder ausschließlich Kohlenwasserstoffe mit drei und mehr Kohlenstoffatomen enthält, und die in Form eines Stoffstroms H in eine destillative Trennung 60 überführt wird. In dieser wird ein Stoffstrom I, der überwiegend oder ausschließlich den oder die Gefrierinhibitoren enthält, die dem Stoffstrom C zugespeist werden sollen, insbesondere Toluol, gebildet. Dieser wird zur Bildung des bereits erwähnten Stoffstroms L optional mit einem oder mehreren entsprechenden Stoffströmen K von der Anlagengrenze vereinigt.

In der destillativen Trennung wird im dargestellten Beispiel eine Reihe weiterer Stoffströme gebildet, die hier zusammengefasst mit M bezeichnet sind. Hierbei handelt es sich beispielsweise um überwiegend oder ausschließlich Kohlenwasserstoffe mit drei und/oder vier und fünf und/oder vier bis sechs und/oder mehr als vier und/oder mehr als sechs und/oder mehr als sieben Kohlenstoffatomen, die einzeln oder als Sammelfraktionen bereitgestellt werden können.

## Patentansprüche

1. Verfahren (100) zur Herstellung von Olefinen, bei dem unter Einsatz eines oder mehrerer stoffumwandelnder Prozesse ein Gasgemisch gebildet wird, das zumindest Methan, Ethan, Ethylen, Kohlenmonoxid und Wasserstoff sowie aromatische Verbindungen, darunter zumindest Benzol, enthält, und das einer Tieftemperaturtrennung (30, 40) zugeführt wird, in welcher es von einem ersten auf ein zweites Temperaturniveau abgekühlt wird, **dadurch gekennzeichnet, dass** das Gasgemisch vor der Tieftemperaturtrennung (30, 40) an einem oder mehreren Gefrierinhibitoren angereichert wird.

2. Verfahren (100) nach Anspruch 1, bei dem als der oder die Gefrierinhibitoren, mit dem oder denen das Gasgemisch vor der Tieftemperaturtrennung (30, 40) angereichert wird, eine oder mehrere Verbindungen verwendet werden, die in dem Gasgemisch bereits enthalten ist oder sind.

3. Verfahren (100) nach Anspruch 1 oder 2, bei der der oder die Gefrierinhibitoren, mit dem oder denen das Gasgemisch vor der Tieftemperaturtrennung (30, 40) angereichert wird oder werden, einen Schmelzpunkt von weniger als -80 °C aufweist oder aufweisen.

4. Verfahren (100) nach einem der vorstehenden Ansprüche, bei dem der oder die Gefrierinhibitoren, mit dem oder denen das Gasgemisch vor der Tieftemperaturtrennung (30, 40) angereichert wird oder werden, aromatische und/oder alizyklische und/oder nichtzyklische Kohlenwasserstoffe umfassen.

5. Verfahren (100) nach einem der vorstehenden Ansprüche, bei dem der oder die Gefrierinhibitoren, mit dem oder denen das Gasgemisch vor der Tieftemperaturtrennung (30, 40) angereichert wird, aus der Gruppe ausgewählt ist oder sind, die Toluol, Ethylbenzol, n-Propylbenzol, Cumol und n-Butylbenzol umfasst.

6. Verfahren (100) nach einem der vorstehenden Ansprüche, bei dem der oder die stoffumwandelnden Prozesse eine oxidative Kopplung von Methan und/oder eine Dampfspaltung kohlenwasserstoffhaltiger Einsätze umfassen.

7. Verfahren (100) nach einem der vorstehenden Ansprüche, bei dem das Bilden des Gasgemischs umfasst, ein Produktgemisch des oder der stoffumwandelnden Prozesse an Kohlendioxid und Wasser abzureichern.

8. Verfahren (100) nach einem der vorstehenden Ansprüche, bei dem die Tieftemperaturtrennung (30, 40) eine Demethanisierung (40) umfasst.

9. Verfahren (100) nach einem der vorstehenden Ansprüche, bei dem das erste Temperaturniveau bei 0 bis 100 °C liegt und bei dem das zweite Temperaturniveau bei -60 °C oder darunter liegt.

10. Verfahren (100) nach einem der vorstehenden Ansprüche, bei dem das Benzol in einem Gehalt von bis zu 2.000 ppm in dem Gasgemisch enthalten ist.

11. Verfahren (100) nach einem der vorstehenden Ansprüche, bei dem der oder die Gefrierinhibitoren, mit dem oder denen das Gasgemisch vor der Tieftemperaturtrennung (30, 40) angereichert wird, in der Tieftemperaturtrennung (30, 40) oder einem sich dieser anschließenden Trennschritt (50) aus dem Gasgemisch, das mit ihm oder ihnen angereichert wurde, oder einer in der Tieftemperaturtrennung (40, 50) oder dem anschließenden Trennschritt (50) gebildeten Fraktion abgetrennt wird oder werden.

12. Verfahren (100) nach Anspruch 11, bei dem unter Verwendung zumindest eines Teils des Gasgemischs ein Komponentengemisch gebildet wird, das neben dem oder den Gefrierinhibitoren, mit dem oder denen das Gasgemisch der Tieftemperaturtrennung (30, 40) angereichert wird, überwiegend oder ausschließlich Kohlenwasserstoffe mit drei und mehr Kohlenstoffatomen umfasst.

13. Verfahren (100) nach Anspruch 12, bei dem der oder die Gefrierinhibitoren, mit dem oder denen das Gasgemisch vor der Tieftemperaturtrennung (30, 40) destillativ aus dem Komponentengemisch oder einem Teil hiervon abgetrennt wird oder werden.

14. Anlage zur Herstellung von Olefinen, die dafür eingerichtet ist, unter Einsatz eines oder mehrerer stoffumwandelnder Prozesse in einer oder mehreren Reaktionseinheiten (10) ein Gasgemisch zu bilden, das zumindest Methan, Ethan, Ethylen, Kohlenmonoxid und Wasserstoff sowie aromatische Verbindungen, darunter zumindest Benzol, enthält, und das Gasgemisch einer Tieftemperaturtrennung (30, 40) zuzuführen, in welcher es von einem ersten auf ein zweites Temperaturniveau abgekühlt wird, **dadurch gekennzeichnet, dass** Mittel bereitgestellt sind, die dafür eingerichtet sind, das Gasgemisch vor der Tieftemperaturtrennung (30, 40) an einem oder mehreren Gefrierinhibitoren anzureichern.

15. Anlage nach Anspruch 14, die zur Durchführung eines Verfahrens nach einem der Ansprüche 1 bis 13 eingerichtet ist.
